# EUROPEAN PATENT SPECIFICATION

(11) **EP 3 131 856 B1**
(45) Date of publication and mention of the grant of the patent: **02.11.2022**
(21) Application number: 15780415.4
(22) Date of filing: 16.04.2015
(51) Int. Cl.: C02F 11/02, C02F 11/04, C12P 3/00, C12P 7/02, C12P 7/18, C12M 1/107, C12M 1/00, B09B 3/00

(54) **THE PRODUCTION OF HYDROGEN AND OTHER GASEOUS OR LIQUID PRODUCTS IN AN ACCELERATED BIOPROCESS**
HERSTELLUNG VON WASSERSTOFF UND ANDEREN GASFÖRMIGEN ODER FLÜSSIGEN PRODUKTEN IN EINEM BESCHLEUNIGTEN BIOLOGISCHEN VERFAHREN
PRODUCTION D'HYDROGÈNE ET D'AUTRES PRODUITS GAZEUX OU LIQUIDES DANS UN BIOPROCÉDÉ ACCÉLÉRÉ

(30) Priority: 16.04.2014 FI 20140114
(43) Date of publication of application: 22.02.2017
(73) Proprietor: Hakalehto, Eino Elias, 70110 Kuopio (FI)
(72) Inventor: Hakalehto, Eino Elias, 70110 Kuopio (FI)
(74) Representative: Tomkinson, Alexandra
(86) International application number: PCT/FI2015/000015
(87) International publication number: WO 2015/158950

(56) References cited:
- WO-A1-2005/118775
- WO-A1-2013/119866
- WO-A2-2011/110731
- WO-A2-2011/110731
- WO-A2-2014/052920
- JP-B2- 5 334 077
- KR-B1- 101 180 869
- US-A1- 2007 048 851
- US-A1- 2012 252 082
- US-B1- 7 232 669
- ZA-A- 200 808 395
- Y. Nakashimada: "Enhanced 2,3-butanediol production by addition of acetic acid in Paenibacillus polymyxa", Journal of Bioscience and Bioengineering, 1 January 2000 (2000-01-01), pages 661-664, XP055023577, DOI: 10.1016/S1389-1723(00)90013-6 Retrieved from the Internet: URL:http://www.jstage.jst.go.jp/article/jb b/90/6/661/_pdf [retrieved on 2012-04-02]
- CHENG K K ET AL: "Improved 2,3-butanediol production from corncob acid hydrolysate by fed-batch fermentation using Klebsiella oxytoca", PROCESS BIOCHEMISTRY, ELSEVIER LTD, GB, vol. 45, no. 4, 1 April 2010 (2010-04-01), pages 613-616, XP026944013, ISSN: 1359-5113, DOI: 10.1016/J.PROCBIO.2009.12.009 [retrieved on 2010-03-05]
- F V Tsvetanova ET AL: "Influence of pH and aeration on 2,3-butanediol production from glucose by Klebsiella pneumoniae G31", Bulgarian Chemical Communications, Volume 46, Number 4, 1 January 2014 (2014-01-01), pages 784-787, XP055423576, Retrieved from the Internet: URL:https://www.researchgate.net/profile/K aloyan_Petrov/publication/286067707_Influe nce_of_pH_and_aeration_on_23-butanediol_pr oduction_from_glucose_by_Klebsiella_pneumo niae_G31/links/5676873b08ae0ad265c54c9d/In fluence-of-pH-and-aeration-on-2-3-butanedi ol-production-from-glucose-by-Klebsiella-p neumoniae- [retrieved on 2017-11-10]
- HANNO RICHTER ET AL: "A Two-Stage Continuous Fermentation System for Conversion of Syngas into Ethanol", ENERGIES, vol. 6, no. 8, 7 August 2013 (2013-08-07), pages 3987-4000, XP055424750, DOI: 10.3390/en6083987
- M. RIVAROLO ET AL: "Hydro-methane and methanol combined production from hydroelectricity and biomass: Thermo-economic analysis in Paraguay", ENERGY CONVERSION AND MANAGEMENT., vol. 79, 1 March 2014 (2014-03-01), pages 74-84, XP055424449, GB ISSN: 0196-8904, DOI: 10.1016/j.enconman.2013.11.044
- KÖPKE M ET AL.: '2,3-Butanediol production by acetogenic bacteria, an alternative route to chemical synthesis, using industrial waste gas.' APPLIED AND ENVIRONMENTAL MICROBIOLOGY vol. 77, no. 15, August 2011, pages 5467 - 5475, XP055104754
- WU K J ET AL: "Simultaneous production of 2,3-butanediol, ethanol and hydrogen with a Klebsiella sp. strain isolated from sewage sludge", BIORESOURCE TECHNOLOGY, ELSEVIER, AMSTERDAM, NL, vol. 99, no. 17, 1 November 2008 (2008-11-01), pages 7966-7970, XP023182691, ISSN: 0960-8524, DOI: 10.1016/J.BIORTECH.2008.03.062 [retrieved on 2008-05-13]
- A K Sayah ET AL: "CO2 Abatement by Methanol Production from Flue-Gas in Methanol Plant", World Academy of Science, Engineering and Technology, 70, 1 January 2010 (2010-01-01), pages 90-93, XP055705511, Retrieved from the Internet: URL:http://citeseerx.ist.psu.edu/viewdoc/d ownload?doi=10.1.1.190.7667&rep=rep1&type= pdf [retrieved on 2020-06-16]
- Perego P. ET AL: "Experimental study of hydrogen kinetics from agroindustrial by-product: Optimal conditions for production and fuel cell feeding", BIOPROCESS ENGINEERING, vol. 19, no. 3, 1 January 1998 (1998-01-01), pages 205-211, XP055809795, Berlin/Heidelberg ISSN: 0178-515X, DOI: 10.1007/s004490050507 Retrieved from the Internet: URL:https://link.springer.com/content/pdf/ 10.1007/s004490050507.pdf>
- Perego P. ET AL: "2,3-Butanediol production by Enterobacter aerogenes: selection of the optimal conditions and application to food industry residues", BIOPROCESS AND BIOSYSTEMS ENGINEERING, vol. 23, no. 6, 15 December 2000 (2000-12-15), pages 613-620, XP055809794, DE ISSN: 1615-7591, DOI: 10.1007/s004490000210 Retrieved from the Internet: URL:https://link.springer.com/content/pdf/ 10.1007/s004490000210.pdf>

## Description

### BACKGROUND

The utilization of microbes in the disintegration of biomasses is known waste treatment technology. In this action, the organic substances contained in the waste materials, change to an altered chemical form as a result of microbe metabolism. A part of the carbon, oxygen, nitrogen, sulfur and hydrogen that originate from the organic material, change their state, for example from solid substances to liquids or from liquids to gases. These, in turn, can react further. On the other hand, nitrogen (N₂) binds to organic material through biological nitrogen fixation in many microbe processes.

In addition to waste materials, microbiological process technology can use, as raw materials, different side streams from manufacturing, agriculture and forestry or other industries and organic material in nature's biomasses. In the plant kingdom, the majority of biomass is cellulose, but for example also hemicellulose, lignin and starch contain significant reserves. A lot of bound chemical energy has concentrated into these reserves. Plant-derived biomass sources are for example wood material, algae, peat, harvest wastes and many food industry side streams.

Organic compounds consist of so-called hydrocarbon backbones. In connection with burning, carbon oxides and water are produced from these. The carbon of hydrocarbons bind into the first mentioned and hydrogen into the latter. The transformation of the organic material of biomasses in a low-oxygen environment produces methane (CH₄), hydrogen (H₂) and carbon dioxide (CO₂). In addition hydrogen sulfide (H₂S) and ammonia (NH₄) are formed. Future energy production and the chemical industry that links to it is often referred to as "hydrogen economy". The separation of hydrogen from water with the help of electric current is used currently amply utilized in the production of hydrogen. In this case the energy balance in a whole is negative as the energy content of electricity is greater than the freed hydrogen. This is caused because wastage cannot totally be avoided. In addition, the production method of the used electricity materially affects the ecology of the produced hydrogen energy.

Although a lot of hydrogen forms in biochemical and microbiological reactions, in its gaseous form it is normally mixed into other gases, such as carbon dioxide, methane, vaporized ammonia, nitrogen sulfide and vaporizing organic compounds (VOC). The latter of these are often also products that can be utilized. An explosion risk also associates with hydrogen (and hydrogen sulfide), and in addition hydrogen sulfide is notably toxic. Corrosion problems brought to production plants with acids and alkali are linked with the formation of hydrogen sulfide and ammonia. The removal of sulfur has successfully been practiced as a known technology in connection with oil drilling and petrochemical industries.

The purification of hydrogen from a mixed gas flow is also known technology. Thus the biotechnical production of hydrogen is industrially possible to implement with current knowledge if the yield and productivity can be raised to a satisfactory level.

Problems also relate to the recovery of hydrogen and especially its safe production especially in or near populated areas, cities and industrial reach areas. Because of this the production facilities for hydrogen have to be designed with care. Specifically this is highlighted because large volumes of hydrogen are formed from often large amounts of waste materials or side streams. On the other hand, in microbiological or biochemical hydrogen production tests the productivity levels have remained fairly low, which is a complicating factor in regards to the economical implementation of the production process. Additionally, in waste treatment, the climate load caused by carbon emission and the further processing of the residual biomass, have to be solved in an economical, safe and ecologically viable way.

Recent public debate has brought up the aspect that in many respects the biomass based waste has transformed into a valuable side stream and that its sufficiency and availability must be secured full-time. Thus the reduction of transport and storage costs of organic waste and other biomass reduce the price of energy and chemicals that can be gained from this side stream. Therefore it is important to arrange, for example, the production of hydrogen near a populated area, industry or agricultural and forestry units where the biomasses form.

In many microbiological fermentation phenomena, hydrogen forms as a result of the action of dehydrogenase enzymes. This reaction is reversible. In the binding of hydrogen to the biomass from water, operative are the hydrolase, i.e. hydratase or hydrase enzymes that bind hydrogen contained in water to organic molecules. The biochemical and microbiological and biotechnical production of hydrogen has not often reached such a level of production that the burning or liquefying of hydrogen gas, and possible other released burnable gases, profitably would require. The solving of this question in regards to energy production is a significant matter in overall economics, especially, if at the same time the release of climate impact causing carbon (and nitrogen) oxides or methane from wastes into the atmosphere can be decreased or eliminated, and the economically and ecologically meaningful use of the residual biowaste can be solved (repository). Thus it is possible to implement energy production in large scale on an economically, energetically and ecologically profitable and sustainable basis when moving towards hydrogen economy, or broader to bioeconomy.

Cheng K K et al: "Improved 2,3-butanediol production from corncob acid hydrolysate by fed-batch fermentation using Klebsiella oxytoca", Process Biochemistry, Elsevier Ltd, GB, Vol. 45, no. 4, April 2010, pages 613-616, XP026944013, ISSN: 1359-5113, DOI:10,1016/J.PROCBIO.2009.12.009 discloses the improved 2,3-butanediol production from corncob acid hydrolysate by fed-batch fermentation using Klebsiella oxytoca. The pH of the cultures was maintained at 6.3, the growth conditions of the bacteria were optimised and an airflow was provided through the culture.

F V Tsvetanova et al: "Influence of pH and aeration on 2,3-butanediol production from glucose by Klebsiella pneumoniae G31", Bulgarian Chemical Communications, Vol. 46, No. 4, 1 January 2014, pages 784-787, XP055423576 discloses that an optimum pH of 6.0 and aeration optimised the 2,3-butanediol production from glucose by Klebsiella pneumoniae G31, together with other optimised growth conditions.

US2007/048851 discloses a method of sustained microbial production of hydrogen gas and carbon dioxide in a bioreactor using Klebsiella oxytoca. The pH was maintained between 3.5-6.0 and the growth conditions of the bacteria were optimised.

Wu K J et al: "Simultaneous production of 2,3-butanediol, ethanol and hydrogen with a Klebsiella sp. Strain isolated from sewage sludge", Bioresource Technology, Elsevier, Amsterdam, NL, Vol. 99, no. 17, 1 November 2008, pages 7966-7970, XP023182691, ISSN: 0960-8524, DOI: 10.1016/J.BIORTECH.2008.03.062 discloses the use of isolating a strain of Klebsiella sp. HE1 from hydrogen producing sewage sludge in order to produce 2,3-BD, ethanol and hydrogen. The isolated strain of bacteria was fully optimised for growth using agar growth plates and growth media without the use of a carrier gas.

Perego P. et al: "2,3-Butanediol production by Enterobacter aerogenes: selection of the optimal conditions and application to food industry residues", Bioprocess and Biosystems Engineering, Vol. 23, No. 6, 15 December 2000, pages 613-620, SP055809794, ISSN: 1615-7591, DOI:10.1007/s004490000210 discloses an optimum pH of 6 is required for Enterobacter aerogenes in the production of 2,3-butanediol. The bacteria were grown in growth media.

Perego P et al: "Experimental study of hydrogen kinetics from agroindustrial by-product: Optimal conditions for production and fuel cell feeding", Bioprocessing Engineering, Vol. 19, no. 3, 1 January 1998, pages 205-211, XP055809795, ISSN:0178-515X, DOI:10.1007/s004490050507 discloses the use of Escherichia coli and Enterobacter aerogenes strains in the optimisation of hydrogen production in batch reactors. The bacteria were grown in growth media.

### DESCRIPTION OF INVENTION

According to the present invention there is provided a microbiological production method for producing a metabolic product in the form of gaseous H2 and/or liquid 2,3-butanediol according to claim 1.

The essential and central feature of the method according to this invention is that the metabolism of microbes as a whole is limited and guided so that the metabolism of the compounds that contain actual organic carbon is restrained or attenuated. Then at cellular level, instead of anabolism or catabolism, so-called overflow metabolism is exploited. The meaning of this action is to exploit the dehydrogenase and hydrase type of enzymes of microbes so that they are able to release hydrogen as gas (dehydrogenase) and respectively bind water (hydrases), and the hydrogen that originates from this water replaces the released hydrogen. Correspondingly, as a result of microbe action, it is possible to get the protons that have formed into the culture media in connection with acid production, to react with the electron flux that forms between the liquid and gas phases so that hydrogen forms in this reaction also. Organic compounds, such as 2,3 -butanediol are often formed as liquid products of the overflow metabolism.

In order to optimize the way that hydrogen is forming and releasing for the purpose of collecting or using it, a carrier gas is led to the biomass either continuously or occasionally. In this case the functional conditions of dehydrogenase enzymes that are contained in produced by microbes can be improved e.g. by restraining the pH decrease and inhibition of the final product.

This gas may be, for example, inert nitrogen, or carbon oxides, or a mixture of the previous. The gas mixture may contain also oxygen, if needed, but the process may also be entirely anaerobic. In order to have the metabolism and evaporation of carbon as carbon dioxide to be restrained at the same time in the said bioprocess, pH control or the modification of other environmental conditions may be used as help so that the conditions are not favorable for the normal anabolic and catabolic metabolism of microbes. The hydrogen that is released in overflow metabolism or similar microbial biochemical activity can be replaced in organic material with water contained in microbes or produced with the help of hydrases.

### Brief description of the drawings

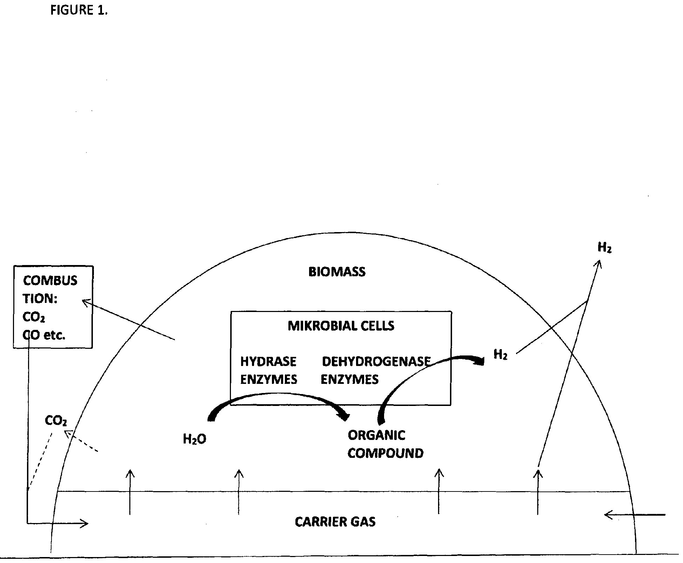

Fig. 1 illustrates the general principle for the liberation of hydrogen from the organic waste or other biomass according to the method of the present invention.
Fig. 2 illustrates the phased utilization of organic waste or other biomass raw material into hydrogen and into further processing. In this example five (5) days lasting "divisional" process model.
Fig. 3 illustrates a solution for hydrogen production inside modern urban setting.
Fig. 4 illustrates E. coli (curves a and b) and Klebsiella mobilis (curves b and c), growth in PMEU Spectrion^{©} equipment in aerobic (curves a and c) and in low oxygen (curves b and d) conditions. Fig. 5 illustrates sugary water derived from sugar industry process, into which water also waters from tank washes were mixed with, were cultivated on the BHI medium in the PMEU Spectrion^{©} equipment. Microbial growth was visible in about 12 h after the onset of the culture.

In low pH (under 5 or under 4,5), the function of dehydrogenases continued actively although the release of gases such as carbon dioxide and methane, that relate directly to the cycle of carbon, decreased from the biomass. Thus the production of hydrogen can be maintained in conditions in which carbon is not released in the same proportion in gaseous form as in more favorable conditions from the point of view of mixed microbe population. It is likely that the release of hydrogen relates from the point of view of microbes to the action with which they attempt to restrain pH decrease.

The production of 2,3-butanediol, that for example *Klebsiella* sp and *Enterobacter* sp and many other microbes, often bacteria that originate from the intestines, actively carry out, is partly associated with the inhibition of pH decrease by action of microbe overflow metabolism. According to our research, this production accelerates in this so-called overflow metabolism when the sugar content of the substrate liquid increases. By changing glucose to 2,3-butanediol, ethanol and gases, the butanediol fermentation of bacteria balances not only the pH but also the osmotic pressure of their habitat.

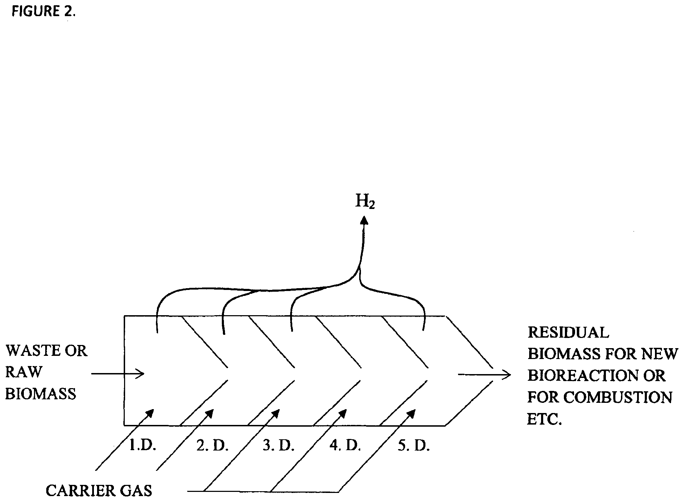

By exploiting the natural or added bacteria or other microbial mixed culture if waste materials (potato waste, mass production waste materials and side streams, wastes from food industry, manure, straw, wood material, peat, waste sludge, community wastes etc.) it is possible to produce a flux of vaporizing hydrogen that is based on the action of dehydrogenase enzymes. As hydrogen evaporates in gaseous form new hydrogen binds from water to the organic material whose release of carbon can be restrained for example by pH control. In practical implementation the facility that receives organic waste or biomass can operate with an batch principle for example so that the waste or other receivable biomass that is produced during one day is treated as one batch which for example during 5 days is transferred in the treatment facility from one department to another (Figure 2). Then the formation of hydrogen and its release into the carrier gas is intensified and controlled e.g. with the help of gas flow, pH and temperature.

Biotechnologically the achieved benefit is that in the process (biomass raw material) the net carbon content remains high, and this can also be maintained e.g. by leading into it carbon dioxide and carbon monoxide, that are released in connection with the burning of dried waste or other biomass, or the exhaust gas from the fermentation process either as such or refined. By proceeding like this, the value of the biomass raw material as a substrate lasts and from it e.g. fuels, chemicals and fertilizers can be produced.

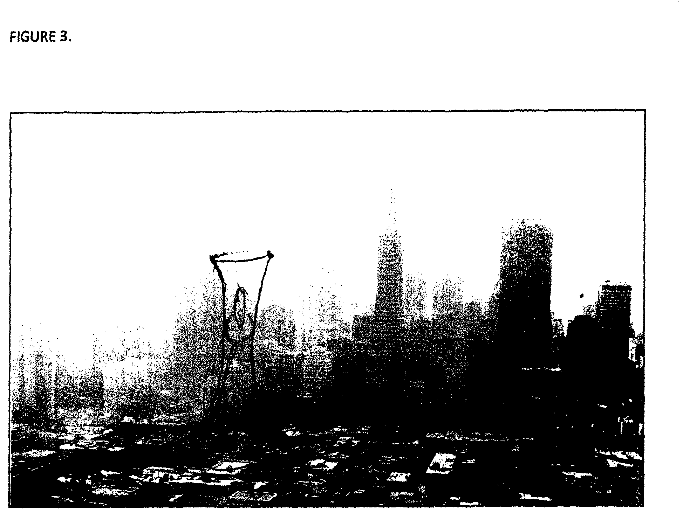

Methanol, which can be exploited as liquid fuel, is achieved when carbon monoxide and hydrogen released from the bioprocess react, or alternatively hydrogen can be liquefied for example to transport fuel. Different methods for the storage of hydrogen have been researched and published (Hirscher 2010). In this case when microbe cultures and their dehydrogenase enzymes are used, the high need for electrical energy, which generally relates to the industrial production of hydrogen, is avoided. In a facility built for this purpose, if wanted, the carbon monoxide of combustion gases and the hydrogen produced by microbes can be led together and the formed methanol can be collected. Hydrogen gas can also be burned in a combustion chamber that is heavily protected on its sides but opens upwards so that possible powerful combustion reactions or explosions channel upwards. Thus built facilities can be raised safely even in the middle of cities in which case sewage waste and possible other wastes can be collected under refinery and power plant towers, for example underground in tanks, process equipment or similar (Figure 3). If necessary, the releasing hydrogen (H₂) can be made to react with carbon monoxide in different combustion gases, in which case methanol is formed. The mixture of "residual" carbon dioxide or carbon monoxide that is released from the bioprocess and nitrogen that acts as carrier gas, can be led again into the liquid, i.e. to the process.

That the hydrogen production of microbes in their metabolism either partially or entirely disconnect from the growth and energy metabolism of the population, is an indication that this gas formation is part of the overflow metabolism whose purpose is to modify the living conditions of the microbes to be more favorable. Respectively, high 2,3-butanediol concentrations are the result of the neutralization of organic acids, specifically the reactions of acetic acid. This phenomena has been noted for example in a mixed culture of *Klebsiella mobilis* and *Escherichia coli* bacteria or in a pure *Klebsiella* culture (Hakalehto *et.al.* 2008, Hakalehto *et.al.* 2010). This way record production levels of 2,3-butanediol from food industry waste have been gained (Hakalehto *et.al.* 2013). In these same reactions also hydrogen has been formed and the forming of hydrogen is not always directly linked to the release of carbon dioxide. The latter gas can also be led back to the culture in which case it stimulates the cell growth of, for example, *E. coli, Klebsiella* and *Clostridium* sp. (Hakalehto 2011a, Hakalehto 2013, Hakalehto 2014). It has been shown that *Clostridium* bacteria can grow even in a 100% CO₂ flow (Hell *et.al.* 2010). With their help, e.g. butanol, ethanol and hydrogen can be produced.

Butanediol can be used as a raw material in the production of synthetic rubbers, plastics, de-icing agents, fibers, cosmetic agents or in drug manufacturing. When refined, butanol is suitable for motor fuel. Liquid hydrogen is a suitable fuel for FCV type vehicles.

In biochemical hydrogen production tests so far the productivity levels have generally been fairly low due to the slow diffusion of the forming gas in sludge, process broth, biomass suspension or other raw material. The products of biochemical reactions easily restrict or prevent their own forming through final product inhibition or similar other metabolic control mechanisms that relate to the reactions of forming hydrogen or other products. In conventional fermentation the production of hydrogen is directly proportional to the emission of carbon and thus to an adequate source of carbon in the culture as well as close to optimal level pH conditions.

However, in a surprising way according to this invention, it is possible to maintain and continue the production of gaseous material, such as hydrogen and liquid material such as 2,3-butanediol at a high level in unfavorable conditions in regards to the anabolism and catabolism of microbes. Then, for example, at a too low or too high pH level, hydrogen can still be released or a too high glucose concentration (osmotic pressure) can be exploited in 2,3-butanediol production. In hydrogen production a requirement is that the used pH is, in regards to the active hydrogenases in the process, a possible condition even if it doesn't allow powerful anabolic or catabolic metabolism. Rinsing with gas which is done to the mentioned biomass with the help of leading into it a carrier gas, whose temperature can advantageously be controlled, releases gaseous hydrogen in previously described conditions without the emission of carbon essentially accelerating. Thus, in a way, carrier gas is used to extract hydrogen from organic waste or other biomass.

Hydrogen is formed in connection to different microbiological fermentation reactions. These are for examplei:
- 2,3-butanediol fermentation
- Acetone-butanol fermentation
- Microbiological degradation of glycerol or other polyalcohols
- Formation of formic acid after which the formic acid disintegrates form hydrogen and carbon dioxide

The formation of hydrogen is at the same time part of a metabolic reaction sequence resulting in the decreasing acidification of the microbes' habitat. Acetic acid, lactic acid, butyric acid, propionic acid and many other organic acids have first formed into the microbes' medium either as a result of their own actions or that of other microbes (Hakalehto et. al. 2008). Then the medium's proton concentration increases. In order to get to form a simultaneous proton and electron flux, resulting in the formation of hydrogen gas, a clear gradient is needed for the same directional movement of both the protons and electrons. This is advantageously achieved by gassing the medium, that is in liquid or suspension form, with a suitable gas, such as nitrogen, or a mixture of nitrogen and carbon oxides. The carrier gas in question can willingly be in a different temperature than the medium. In this case the direction of both the protons and electrons directs towards the flowing gas and they fuse into hydrogen gas (H₂) on interfaces. As a consequence of this a larger proportion of hydrogen also is released than in common fermentation reactions. By using a method according to this invention these fermentation reactions can be restricted for example by lowering or raising the pH over or under normal levels without the production of hydrogen essentially decreasing. This makes possible significant energy production from biomasses and organic wastes without carbon escaping relatively speaking for example as carbon dioxide. Hydrogen can also be separated from other releasing gases, such as carbon dioxide, which can be returned to the medium with the carrier gas. Respectively also other gases that are released during the burning of biomass or organic waste or other fuel, can be fused to the carrier gas. In this case most fermentation reactions can be stopped or restricted with a high or low temperature without essentially decreasing the release of hydrogen gas. This gas forms in extreme conditions further as a result of the enzyme activity of microbes, and respectively new hydrogen bind from water also enzymatically to organic material.

That the intracell dehydrogenases of bacteria often perform most actively when the cells are in conditions of either low or high pH (Taguchi et. al. 1982, Höhn-Bentz & Radler 1978) demonstrates that their activity is directed to balance and protect the cell's living conditions. For example, the thermophilic *Thermus* sp. bacteria's L-lactate dehydrogenase operates this way in optimal cultures whose temperature was 80 °C and pH 4,5 (Taguchi et. al. 1982). Thus the extreme conditions of the bioreactor (fermentor) can be utilized according to this invention to produce hydrogen from wastes or other biomasses. In low or high pH levels metabolism based on carbon compounds attenuates but the evaporation of hydrogen balances the changed pH in the medium after the forming of acid or alkali. Thus the pH of the bioreactor can be let to lower for example to the pH value of 4,5-5 or 4,0-4,5, in which case the bacteria growth normally attenuates but the production of hydrogen remains at a high level. Consequently the growth and fermentation of bacteria can be restricted either with high or low temperature.

A method according to the present invention can be utilized for example in the transformation of organic sewage sludge or landfill waste, agricultural waste or different industrial wastes to energy and chemicals. Thus, according to our measurements, from organic waste with a few percent of solids several percent of hydrogen can be obtained by releasing this to the carrier gas that flows through the liquid and biomass. Typically utilized agricultural wastes are manure and different harvesting wastes. Of industrial wastes for example dairy industry, potato- and corn, sugar and convenience food industry's side streams and wastes, as well as forestry industry side streams and wastes can be exploited.

In connection with production studies that we have done on 2,3-butanediol since 2007, it has been noted that when butanediol was produced from mass or potato industry wastes, the production of gas decreased in this fermentation while the production of butanediol grew. Results from these studies have partially been published e.g. in international patent applications (PCT/FI2008/000001, PCT/FI2011/00016) and later in literature (Hakalehto et.al. 2008, Hakalehto et.al. 2013).

The decrease of gas production mentioned above relates specifically to the decrease of the formation of carbon dioxide that is being released in fermentations (and partially in cell respiration). Correspondingly, more hydrogen has formed as a byproduct of 2,3-butanediol fermentation than at a lower 2,3-butanediol production level. This has not nevertheless necessarily shown up in visual observation because hydrogen, while vaporizing, has diffused through the biomass and bound back to the biomass and partly diffused through it, and its' release has not become visible as an increased formation of bubbles. In our earlier studies the decrease of gas bubble formation has been linked with the increase of 2,3-butanediol formation (PCT/FI2011/00016). This can thus possibly and likely be explained at least as a decreased carbon dioxide production and does not necessarily indicate the amount of produced hydrogen.

Using a method according to this invention, hydrogen that has formed from a biomass with the help of microbes and their enzymes, can be made to transfer into the gas flow and the bubbles that flow through the biomass. The concentration differences of the electrons and protons between the gas bubbles and the biomass accelerate the corresponding electron and proton flow which likely causes electrical phenomena e.g. at the phase interfaces thus accelerating the formation of hydrogen. Once 2,3-butanediol has formed in the above mentioned way, without a significant increase of cell concentration of bacteria forming it, this metabolism is clearly an overflow type (Table 1 and 7).

**Table 1. The K and KS bacteria content of the cultures at time point 25.5 hours. The bacteria contents of E. coli- and K. mobilis were surprisingly similar. These plate results also indicate that the accelerated reactions in the KS culture that relate to the plentiful formation of 2,3-butanediol were not in any way part of the anabolic route, which in this case is contradictory to the current theory (Johansen et.al. 1975, Yu & Saddler 1983).**

| Culture/bacterial concentration | K | KS |
|---|---|---|
| E. coli | 3 x 10 exp 7 | 2.4 x 10 exp 8 |
| K. mobilis | 6 x 10 exp 7 | 2.5 x 10 exp 8 |

In this case bacterial cells perform metabolism reactions to compensate, with the help of its metabolism, other otherwise unfavorable development around the cells, such as the decrease of pH or the increase of osmotic pressure (Hakalehto et.al. 2008; Hakalehto et.al. 2010). Then the acetic acid formed by other bacteria incorporates by the action of klebsiellas into 2,3-butanediol and hydrogen is released. When glucose with a carbon-13 label, added to the medium, was given to bacteria, the label was found in the carbonyl carbon of acetate in respect of the 2-carbon of glucose whereas in respect of the 3-carbon of glucose the label was found in both the carbonyl and methyl carbons of acetate. This indicates that acetate is formed as a result of the action two different metabolic routes.

On the basis of prior knowledge (Kluyver 1956), this indicates that acetate binds to 2,3-butanediol. Thus the conversion of glucose into butanediol both lowers osmotic pressure and slows the pH decrease. In order that *Klebsiella* sp. and other bacteria that are capable of butanediol fermentation to perform said fermentation, they need reducing metabolic power. This is gained from the cell's energy reserves such as polyhydroxybutyrate and from hydrogen that has been released from water and enzymatically bound into organic substances. The formation of 2,3-butanediol from hydrogen may happen through the following hypothetical reactions:

According to previous studies, the synthesis route of 2,3-butanediol from pyruvate takes place through acetolactate and acetone to butanediol. There are two enzyme system routes in this action: the pH8 anabolic and pH6 catabolic route. As acidity increases in its surrounding, the microbe cell strives to avoid excessive formation of acid, in which case the catabolic route begins to act, and instead of acetolactate transforming into branched-chain amino acids (leucine, valine), catabolic acetoin begins to form of it in low pH. From this butanediol is formed and the pH increases. Klebsiellas, enterobacteria (= aerobacteria) and others thus regulate their own conditions and environment in regards to the pH in the intestines. Coliforms and other acid generators benefit also from this. In addition there are those species that are capable of interchanging from acid formation to butanediol and vice versa (for example *Serratia* sp., *Aeromonas* sp.). These last mentioned are not normally the dominating flora of the intestinal tract while colibacteria and klebsiellas are.

Hypothesis: must all butanediol form through three carbon pyruvate? Why couldn't an organism split energetically more advantageously six carbon glucose into butanediol (through acetoin) and ethanol. This kind of a metabolism route might be possible! How? Is there possibly a parallel metabolism route for the route that begins from pyruvate? The key question is: does all butanediol always form via the above mentioned route that is linked to branched-chain amino acid synthesis, or is there some other route existing? In the table below, based on earlier study, is presented an understanding of the formation of substances in mixed acid fermentation and 2,3-butanediol fermentation.

**Table 2. (Butylene glycol = 2,3-butanediol) Mixed acid vs. butylene glycole fermentation products in total moles of product per 100 moles of glucose fermented in mixed acid fermentation and in butylene glycol fermentation, and thus in e.g. E. coli and Klebsiella sp., respectively. Based on data from Stanier, R.Y., Doudoroff, M., and Adelberg, E.A. The Microbial world, p.582. Prentise Hall, Englewood Cliffs, N.J., 1970 (According to Sonnenwirth, 1973).**

| | Product | Mixed acid fermentation | Butylene glycol fermentation |
|---|---|---|---|
| | - Carbon dioxide | 88 | 172 |
| | - Hydrogen | 75 | 35 |
| | - Formic acid | 2.5 | 17 |
| | - Acetic acid | 36 | 0.5 |
| | - Lactic acid | 79 | 3 |
| | - Succinic acid | 11 | 0 |
| | - Ethyl alcohol | 50 | 70 |
| | - 2,3-Butylene glycol | 0 | 66 |
| | | | |
| Total moles of acids produced | | 129 | 20 |

Metabolic reactions:
1. pyruvate CH₃COCOOH + TPP -> "Active acetaldehyde" [CH₃CHO]^{∗}TPP + formate HCOOH
2. Formate becomes hydrogen 1 molecule and carbon dioxide also 1 molecule
3. according to theory active acetaldehyde reacts with another pyruvate and acetolactate is formed (this reaction thus competes with the formation reaction of anabolic branched-chain amino acids)
4. acetolactate becomes acetoin + CO₂ (2 molecules!)
5. 2 H is needed to get butanediol from acetolactate

Comments:
- where does the ethanol come from that (according to the table) forms as much as butanediol?
- according to the table below the substances would be in the following proportion:

**Table 3.**

| Product | Ratio | Butylene glycol fermentation |
|---|---|---|
| - Carbon dioxide | **10** -> 8 | 172 |
| - Hydrogen | 2 | 35 |
| - Formic acid | 1 | 17 |
| - Acetic acid | | 0.5 |
| - Lactic acid | | 3 |
| - Succinic acid | | 0 |
| - Ethyl alcohol | 4 | 70 |
| - 2,3-Butylene glycol | 4 | 66 |

- so from 10 CO₂ one would get 2 hydrogen, 4 butanediol, 4 ethanol and 1 formic acid
- "according to formula" from one formed butanediol one would get 1 formic acid
- because according to theory one formic acid becomes one hydrogen, this means that 3/4 of formic acid (4 x 17 = 68) would be converted into hydrogen and carbon dioxide, ¼ would not have time to react by the measurement time, instead it would become measured as formic acid (in this case there is as much formic acid as butanediol which equals with the theory)
- there should be 7/3 times as much butanediol as hydrogen which is about 87 (half of the measured amount), from this we can get the ration for CO₂ as 8, not 10
- this means that according to traditional theory from one glucose should form, through acetolactone, 2 butanediol and 4 carbon dioxide
- there should be half less of butanediol than carbon dioxide would form based on the above mentioned calculation, which means 2 (there is 4 !)
- there is double the amount of ethanol because according to the above mentioned theory it does not form from the same glucose as butanediol, this means butanediol 4-2=2, ethanol 4, carbon dioxide 8-2x2=4

**Table 4.**

| Product | Ratio | Butanediol fermentation (mol per mol of glucose) | Ratio as per theory |
|---|---|---|---|
| CO₂ | 10 -> 8 | 172 | 4 |
| H₂ | 2 | 35 | 2 |
| HCOOH | 1 | 17 | 1 |
| EtOH | 4 | 70 | 0 |
| 2,3-Butanediol | 4 | 66 | 2 |

From this remains 4 carbon dioxide, 4 ethanol and 2 butanediol.
- so what is the reaction in which butanediol, ethanol and carbon dioxide form in the ratio 2:4:4 which is 1:2:2 from glucose?
- table of the ratio of different atoms in glucose (Glu), butanediol (BD), ethanol (EtOH) and carbon dioxide (CO₂)

**Table 5.**

| | Glu | BD | EtOH | CO₂ |
|---|---|---|---|---|
| H | 12 | 8 | 6 | |
| C | 6 | 4 | 2 | 1 |
| O | 6 | 2 | 1 | 2 |

- from this follows:

**Table 6.**

| | Glu | BD | 2 EtOH | 2CO₂ | Extra atoms in products compared with Glu |
|---|---|---|---|---|---|
| H | 12 | 8 | 12 | | 8 |
| C | 6 | 4 | 4 | 2 | 4 |
| O | 6 | 2 | 2 | 4 | 2 |

- the remain is 4C, 20, 8H which is the compound C₄H₈O₂, what is it? Answer: butyrate (butyric acid)
- from the previous follows that the reaction formula: Glu+butyrate => BD + 2EtOH + 2CO₂ would be valid in this case
- in every case the substances are thus "equal"
- thus from the table below the thought can be led that in a way described by the theory in the beginning, from two glucose, of which one breaks down first into 2 x pyruvate according to traditional formula and the other reacts with butyric acid, would form 2 butanediol, 2 ethanol and 4 carbon dioxide - which means that half of the butanediol forms via the traditional route and the other half via some other route together with 2 ethanol and 2 carbon dioxide
- so where does butyrate come from?
- The energy storage material of almost all bacteria is POLYHYDROXYBUTYRATE (PHB). It is a polymer of butyric acid that they store into their cells.
- certain bacteria (the ones that perform the butanediol fermentation) thus use, when the acidity increases, their energy storage to maintain the viability of their environment, especially in relation to the pH and osmotic pressure, in other words the neutralize and balance their environment and their intra-cellular milieu
- then when the pH increases they will fill their PHB storage again
- stoichiometrically it would fit that from four glucose and two PHB butyric parts the klebsiellas, enterobacteria etc. butanediol fermentation performers produce the 4 butanediol, 4 ethanol, 8 CO₂ and 2 hydrogen (plus one formic acid that possibly does not have time to react to hydrogen and carbon dioxide).
   - The needed butyrate (for the forming of 2,3-butanediol) can presumably originate also from the metabolism of other bacteria, but as an intra-cell source PHB is the likely one.

The microbes in the rumen of a cow make use of the simple organic compounds in the rumen digesta even though the digesta itself does not comprise high concentrations of free monosaccharides or oligosaccharides etc., which would allow rapid reaction and high productivity. Nevertheless, by slow circulation of hydrolyzable polymers in the digesta, it is possible to keep a continuous high level of small molecule compounds, which enables an increase in the productivity of the bioreaction.

The hydrogen that the microbes discharge from the biomass is created by dehydrogenase enzymes, and the same enzymes bind new hydrogen from water to the organic compounds. In this way a continuum is created: a flow of discharging hydrogen gas, into which protons from the barrier layers of cell membranes and reactions that take place on the barrier layers of gas-liquid phases bind. This flow/stream of hydrogen formation from e.g. rumen digesta in the waste matter of a slaughterhouse, or livestock manure or other biomass waste is a surprising phenomenon because simultaneously the level of general metabolism in the cells decreases.

As various research studies have suggested (Kluyver 1956, Hakalehto & Hänninen 2012) CO₂ binds in heterotrophically to biomass, which sets into action a chain of reactions at the cell level. This binding of carbon dioxide accelerates the metabolism of microbes.

The bacteriological research carried out applying PMEU equipment (Hakalehto 2006, Hakalehto et al. 2009, Pesola & Hakalehto 2011, Hakalehto 2011a) have shown that the leading or conducting of gas into the fermentation broth accelerates the growth and metabolism of microbes. In the case of aerobic bacteria this can be partly explained in that the dissolved oxygen, which most commonly is the restrictive factor, diffuses more quickly for the use of cells.

However, in anaerobic fermentation reactions, the conduction of metabolism products - such as hydrogen (H₂) - away from the cells is of greater importance. As a result, reducing capacity disappears from the cultivations, so new capacity must be developed when water reacts with the mineral substrate. The dehydrogenase enzymes have a decisive position in the discharging of hydrogen. As the result of the function of hydrase enzymes, water is bound to the biomass and in this manner the hydrogen storages of organic molecules restock.

The PMEU-technology (Portable Microbe Enrichment Unit) has been developed to accelerate the growth, the initiation of growth and the metabolism of microbes (Hakalehto & Heitto 2012, Hakalehto 2013). The aim has been to develop the methods of microbe research more sensitive and more reliable than they have been, as well as to achieve results faster than before. At the same time, the range of hygiene monitoring methods can be diversified. PMEU accelerates also e.g. the transformation of bacterial spores into usual vegetative cells (Mentu et al. 2009).

PMEU Spectrion^{®} is a portable microbe enrichment unit, which is equipped with an online detector and remote tracking capabilities for measuring relative turbidity. The inside temperature of the device can be adjusted very precisely at the accuracy of 1/10 of a Celsius degree, and the optimal growth of microbes is ascertained by feeding air or gas into the sample to assist the miscibility of nutrients and to accelerate metabolism. In this way, the user of the enrichment unit can create various "protocols" for detecting the specific microbes one chooses.

When using a general medium as many bacteria as possible can be given a boost of maximal growth in the beginning of the cultivation. In this case it is very important to be able to follow on-line - minute by minute - the growth of the PMEU Spectrion^{®} cultivation, and the growth diagrams are saved into the memory of the device. The diagrams can be examined with the help of the remote use function/properties, e.g. via the Internet, and the device can be adjusted also using the remote control (Hakalehto 2010, Hakalehto 2011a/c). The use of PMEU device has been studied and validated in various research projects regarding the environment and water resources (Heitto et al. 2009, Pitkänen et al. 2009, Wirtanen & Salo 2010).

### Example 1

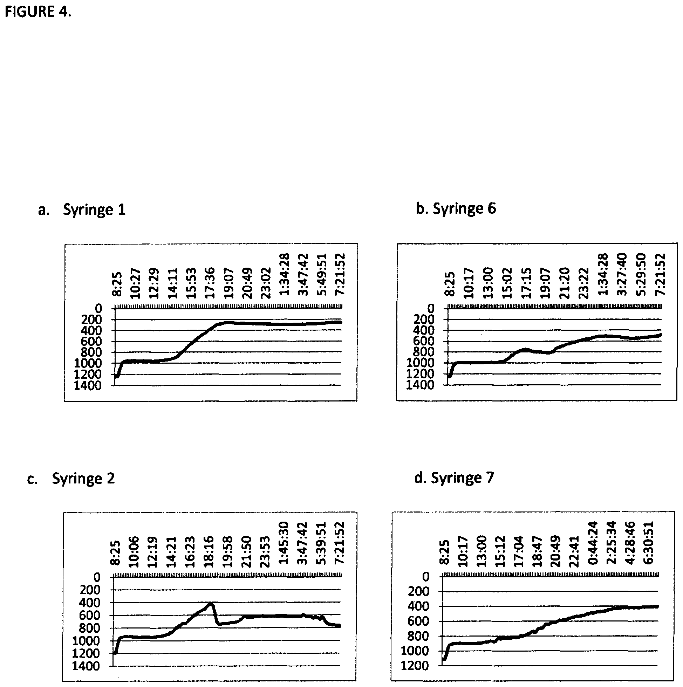

*Escherichia coli* and *Klebsiella mobilis* bacteria were cultivated in cultivation syringes In the PMEU Spectrion^{®} at the temperature of +37 °C. The THG-broth (tryptone-yeast extract-glucose) was used as nutrient medium. In cultivation syringes 1 and 6 there was E. *coli,* and in syringes 2 and 7 there was *Klebsiella.* Syringes 1 and 2 were cultivated in aerobic conditions, and syringes 6 and 7 with a gas mixture (N₂, CO₂, O₂), where the amount of oxygen was reduced. In the example of performance 1 (Figure 4) the metabolism of the *E. coli* bacterium decreased in the reduced oxygen condition when the glucose was used up (4 b.), whereas the function of the Klebsiella bacteria continued undisturbed in the reduced oxygen condition (4.d.) at a speed similar to that of the aerobic cultivation (4.c.) when using a/the carrier gas. With the PMEU it is possible also, if chosen, to study aerobic and anaerobic bacteria simultaneously, which has been ascertained/verified also in previous studies concerning the cultivation of *Salmonella*bacteria (Hakalehto ym. 2007).

### Example 2

This research studied the growth rates of mesophilic bacteria from various sugar mill process samples using the PMEU Spectrion^{®}. The aim was to gain new information of the control of microbe risks and find means/methods of accelerating the hygiene control of processes.

Samples for the PMEU Spectrion^{®} cultivations were taken from the self-monitoring samples of various stages of sugar manufacturing process. Samples were taken from the warm tap water storage tank (VL), from the sugar water of the diluted process, into which also waters from the cleansing of the tanks are mixed (VM), from sugar solutions that have passed through the filters (cp1-ccp6), from the raw material for the refined sugar and syrup, from the boiled juices (1K ja 2K), from the feeder reservoir for the evaporator (40 ga 14), from the pump of the feeder reservoir for the evaporator (43 da 6), from the liquid syrup of container 12 (ga 12) before and after heating, from the liquid syrup of the standardization/conditioning container (round 1, ga 1), from the outgoing liquid syrup (bulk ga 1), and from the "collection tank 6" (43 ga 6), which contains fractions e.g. cleansing waters and syrup waste used as raw material for syrup.

The specimen were cultivated using the PMEU Spectrion and the THG or BHI nutrient broth the temperature being + 30 °C with the exception of one sample take when the cultivation temperature was + 50 °C.

Dish cultivations (mesophilic bacteria in plate count dishes) were carried out using corresponding samples at the microbiological laboratory of the sugar mill.

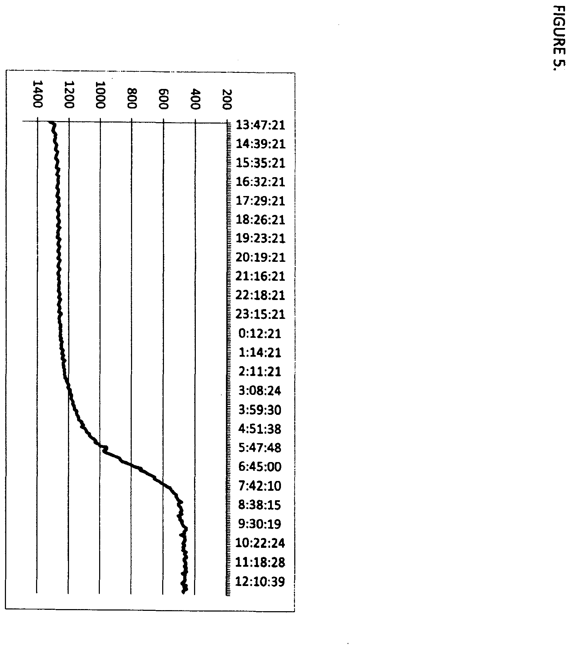

The results of the Spectrion cultivation were presented as diagrams representing the relative turbidity of the samples (see example in Figure 5).

The research demonstrates that the PMEU Spectrion is a very suitable tool for determining the presence of mesophilic bacteria in process samples from a sugar mill industrial plant. When the Spectrion was used and the temperature was + 30 "C, the growth of microbes was detected in 24 hours in all samples, which with the plate count method had the microbe density of 2 - 600 cfu/ml (2 days) as well as in one sample, in which the plate count was 0 cfu/ml. According to the test data, it is possible to suggest that, if growth is not observed in the PMEU Spectrion within 24 hours, mesophilic bacteria do not exist in the studied sample volume.

The PMEU Spectrion is suitable also for the cultivation of sporogenous bacteria which occur in the manufacturing process of sugar (Mentu et al. 2009). The use of high temperatures accelerates the pace of growth which enables the information on the existence of lots of spores in the sample to be obtained as early as during the same working day.

The PMEU method is suitable for microbiological research of samples containing sugar and glucose in biotechnological industry.

Based on previous results, the cultivation results received using the PMEU method have in many cases given outstandingly more reliable quantitative cultivation results than the traditional plate count methods (Hakalehto 2010, Hakalehto 2011a).

On the basis of the PMEU technology, it is possible to develop approaches and methods which save time and effort. It is also possible to add to the method the intensified hygiene control of surfaces, if necessary (Hakalehto, 2006; Hakalehto, 2010). If effectiveness and the shortening of detection times of individual bacteria cells to a few hours is the goal, it is possible to utilize gas detection in bacteria cultivation (Hakalehto et al. 2009).

### Example 3

When growing the *Klebsiella* bacterium on a medium with a glucose concentration we studied the incorporation of carbon-13-marker with acetate and 2,3-butanediol and other compounds, e.g. ethanol, on the one hand, as well as the ratios of the amount of acetate, 2,3-butanediol and ethanol formation in anaerobic conditions on the other hand. The NMR spectrum was recorded with a Bruker Avance DRX 500 spectrometer (Bruker BioSpin GmbH, Germany) the working frequency of which is 500.13 MHz. The samples were prepared for the NMR measurements by adding 50 µl (microlitre) of regulator to 450 µl of extract (1.5mM KH₂PO₄/D₂O, pH 7.4, 2mM NaN₃, 0.1% TSP). The proton NMR spectrum was obtained by using the standard Bruker pulse spectrum NOESYGPPR1D, which is a pre-saturation test for water discharge, which is combined with the first addition of the NOESY pulse sequence, at the spectrum width of 10 kHz, with 64k data points and 64 scannings, giving the digital resolution of 0.15 Hz. The carbon-13 spectra of the samples from the marker tests was measured using the spectrum width of 30 kHz with 128k data points and 96 scannings, giving the digital resolution of 0.23 Hz. The TSP was used as an internal reference. The temperature of the sample was 300 K.

Table 7. Proportions of acetate, ethanol and 2,3-butanediol in tests where glucose was added in high concentrations to a *Klebsiella* cultivation in anaerobiosis. Also the pH of these cultures was controlled during cultivation. It is to be noticed that these numeric values do not represent any absolute values, instead they are relative values. When the acetate had been used up (in culture K), this was an indication that also the amount of exploitable sugars in the nutrient medium was deficient/insufficient. At the point of time 21.5 hours, sugar has increased slightly. This is apparently due to enzymatic hydrolysis.

| Point of Time (hours) | | Culture/Cultivation | Acetate | Ethanol | 2,3-butanediol |
|---|---|---|---|---|---|
| | 0 | C2 | 1 | 0.87 | 0.78 |
| | | C3 | 1 | 0.87 | 0.83 |
| | | | | | |
| | 2 | C2 | 1 | 1.45 | 1.45 |
| | | C3 | 1 | 0.97 | 0.92 |
| | | | | | |
| | 4 (before addition of glucose and | C2 | 1 | 0.53 | 0.20 |
| | acetate) | C3 | 1 | 0.54 | 0.23 |
| | | | | | |
| | 5.5 (after | C2 | 1 | 0.37 | 0.05 |
| | additions) | C3 | 1 | 0.23 | 0.07 |
| | | | | | |
| | 16.5 | C2 | 0 | 0.88 | 0.64 |
| | | C3 | 1 | 2.36 | 3.12 |
| | | | | | |
| | 22 | C2 | 1 | 1.31 | 1.21 |
| | | C3 | 1 | 12.71 | 19.15 |
| | | | | | |
| | 24 | C2 | 1 | 2.29 | 4.78 |
| | | C3 | 1 | 9.16 | 9.33 |
| | | | | | |
| | 25 | C2 | 1 | 2.67 | 4.02 |
| | | C3 | 1 | 4.35 | 5.16 |
| | | | | | |
| | 26 | C2 | 1 | 2.61 | 3.62 |
| | | C3 | 1 | 3.62 | 4.94 |

### REFERENCES

Hakalehto E (2006). Semmelweis' present day follow-up: Updating bacterial sampling and enrichment in clinical hygiene. Review Article. Pathophysiology 13: 257-267.

Hakalehto E (2008). Biotechnical and microbiological production method and equipment. Patent Application PCT/FI2008/000001.

Hakalehto, E (2010). Hygiene monitoring with the Portable Microbe Enrichment Unit (PMEU). 41st R3 -Nordic Symposium. Cleanroom technology, contamination control and cleaning. Espoo, Finland, May 2010. VTT (State Research Centre of Finland) Publications 266.

Hakalehto E (2011a). Simulation of enhanced growth and metabolism of intestinal Escherichia coli in the Portable Microbe Enrichment Unit (PMEU). In: Rogers MC, Peterson ND (eds.) E. coli infections: causes, treatment and prevention. Nova Science Publishers, Inc, New York, USA, pp. 159-175.

Hakalehto E (2011b). Method and apparatus for accelerating biotechnical reaction and production. Patent Application PCT/FI2011/00016.

Hakalehto E (2011c). Antibiotic resistance traits of facultative Enterobacter cloacae strain studied with the PMEU (Portable Microbe Enrichment Unit). In: Mendez-Vilas A (ed.) Science against microbial pathogens: communicating current research and technological advances. Microbiology Series no 3, vol 2. Formatex Research Center, Badajoz, Spain, pp. 786-796.

Hakalehto E (2013). Interactions of Klebsiella sp. with other intestinal flora. In: Pereira LA, Santos A (eds.) Klebsiella infections: Epidemiology, pathogenesis and clinical outcomes. Nova Science Publishers, Inc, New York, USA, pp. 1-33.

Hakalehto E (2014). Enhanced microbial process in the sustainable fuel production. In: Junyue Y (ed.) Handbook of Clean Energy Systems. Wiley, USA. Accepted manuscript.

Hakalehto E, Heitto L (2012). Minute microbial levels detection in water samples by Portable Microbe Enrichment Unit Technology. Environment and Natural Resources Research 2: 80-88. Hakalehto E, Hänninen O (2012). Gaseous CO2 signal initiate growth of butyric acid producing Clostridium butyricum both in pure culture and in mixed cultures with Lactobacillus brevis. Can J Microbiol 58: 928-931.

Hakalehto E, Pesola J, Heitto L, Närvänen A, Heitto A (2007). Aerobic and anaerobic growth modes and expression of type 1 fimbriae in Salmonella. Pathophysiology 14: 61-69.

Hakalehto E, Humppi T, Paakkanen H (2008). Dualistic acidic and neutral glucose fermentation balance in small intestine: Simulation in vitro. Pathophysiology 15: 211-220.

Hakalehto E, Pesola J, Heitto A, Bhanj Deo B, Rissanen K, Sankilampi U, Humppi T, Paakkanen H (2009). Fast detection of bacterial growth by using portable microbe enrichment unit (PMEU) and Chempr0100i° gas sensor. Pathophysiology 16: 57-62.

Hakalehto E, Hell M, Bernhofer C, Heitto A, Pesola J, Humppi T, Paakkanen H (2010). Growth and gaseous emissions of pure and mixed small intestinal bacterial cultures: Effects of bile and vancomycin. Pathophysiology 17: 45-53.

Hakalehto E, Jääskeläinen A, Humppi T, Heitto L (2013). Production of energy and chemicals from biomasses by micro-organisms. In: Dahlquist E (ed.) Biomass as energy source: resources, systems and applications. CRC Press/Balkema, Taylor & Francis Group, Leiden, The Netherlands, pp. 235-262.

Heitto L, Heitto A, Hakalehto E (2009). Tracing wastewaters with faecal enterococci. A poster. Second European large lakes symposium. 10-14 August, Norrtälje, Sweden.

Hell M, Bernhofer C, Huhulescu S, Indra A, Allerberger F, Maass M, Hakalehto E (2010). How safe is colonoscope-reprocessing regarding Clostridium difficile spores? J Hosp Inf 76: 21-22.

Hirscher M (ed.) (2010). Handbook of hydrogen storage. Wiley-VCH Verlag GmBH & Co. KGaA, Weinheim.

Höhn-Bentz H, Radler F (1978). Bacterial 2,3-butanediol dehydrogenases. Arch Microbiol 116: 197-203.

Johansen L, Bryn K, Stormer FC (1975). Physiological and biochemical role of the butanediol pathway in Aerobacter (Enterobacter) aerogenes. J Bacteriol 123: 1124-1130.

Kluyver AJ (1956). Microbial metabolism; further evidence for life's unity. In: Kluyver AJ, van Niel CB (eds.) The microbe's contribution to biology. Harvard University Press, Cambridge, Massachusetts, US, pp. 31-72.

Mentu JV, Heitto L, Keitel H, Hakalehto E (2009). Rapid microbiological control of paper machines with PMEU method. Paperi ja Puu (Paper and Timber) 91: 7-8.

Pesola J, Hakalehto E (2011). Enterobacterial microflora in infancy - a case study with enhanced enrichment. Indian J Pediatr 78: 562-568.

Pitkänen T, Bracker J, Miettinen I, Heitto A, Pesola J, Hakalehto E (2009). Enhanced enrichment and detection of thermotolerant Campylobacter species from water using the Portable Microbe Enrichment Unit (PMEU) and realtime PCR. Can J Microbiol 55: 849-858.

Ohta T (1982). Taguchi H, Yamashita M, Matsuzawa H,

Heat-stable and fructose 1,6-bisphosphate-activated L-lactate dehydrogenase from an extremely thermophilic bacterium. J Biochem 91: 1343-1348.

Wirtanen G, Salo S (2010). PMEU-laitteen validointi koliformeilla (Validation of PMEU equipment with coliforms). Report VTT-S-01705-10, VTT Expert Services Oy, Espoo, Finland.

Yu EKC, Sadler JN (1983). Fed-batch approach to production of 2,3-butanediol by Klebsiella pneumoniae grown on high substrate concentrations. Appl Environ Microbiol 46: 630-635.

## Claims

1. A microbiological production method for producing a metabolic product in the form of gaseous H₂ and/or liquid 2,3-butanediol, said method comprising the steps of:
a) introducing a carrier gas into a raw waste biomass material, said biomass material containing *E*. *coli* and *Klebsiella mobilis;* and wherein the carrier gas conducted into the raw biomass material is an inert gas, nitrogen, carbon dioxide, carbon monoxide, or a mixture of these gases; **characterised in that** said method further includes the steps of
b) providing a pH level of the biomass material of under pH 5 to minimize metabolism of carbon containing compounds in the biomass material and to minimize loss of gaseous carbon in the biomass material;
c) producing a metabolic product in the form of gaseous H₂ and/or liquid 2,3-butanediol from the biomass material in proposition to time and volumetric units without optimizing actual cell growth metabolism within the biomass material;
d) releasing at least a first portion of the metabolic product to a combustion process to produce a combustion product, and introducing the combustion product into the carrier gas prior to introduction into the raw waste biomass material; and
e) capturing at least a second gaseous portion of the metabolic product containing hydrogen.

2. The method according to claim 1, **characterized in that** the microbiological production method is a process selected from an aerobic, a microaerobic or an anaerobic process.

3. The method according to claim 2 further comprising the step of controlling at least one unbeneficial condition by adding to said biomass at least one of glucose, a bacteria or a microbial culture, wherein the unbeneficial condition is selected from the group consisting of reduced pH, increased pH, and osmotic pressure.

4. The method according to claim 3, further including the step of exploiting microbial overflow metabolism for accumulating the metabolic product, or for accelerating liberation of the metabolic product.

5. The method according to claim 1, further including the step of adjusting the temperature of the carrier gas prior to introduction into the raw biomass material.

6. The method according to claim 1, further including the step of concentrating the metabolic product into one selected from the group of a burnable energy source, a motor fuels and a chemical industry raw material.

7. The method according to claim 1 further comprising the step of reacting the hydrogen after step e) with carbon monoxide to create methanol.

8. The method according to claim 1 further comprising the step of conducting the hydrogen from step e) into an incineration tower.

## Patentansprüche

1. Mikrobiologisches Herstellungsverfahren zur Erzeugung eines Stoffwechselprodukts in Form von gasförmigem H₂ und/oder flüssigem 2,3-Butandiol, wobei das genannte Verfahren die folgenden Schritte beinhaltet:
a) Einführen eines Trägergases in ein Roh-Biomassematerial aus Abfällen, wobei das Biomassematerial *E. coli* und *Klebsiella mobilis* enthält; und wobei das in das Roh-Biomassematerial eingeführte Trägergas ein Inertgas, Stickstoff, Kohlendioxid, Kohlenmonoxid oder ein Gemisch dieser Gase ist; **dadurch gekennzeichnet, dass** das genannte Verfahren ferner die folgenden Schritte umfasst:
b) Bereitstellen eines pH-Wertes des Biomassematerials von unter 5, um den Stoffwechsel von kohlenstoffhaltigen Verbindungen in dem Biomassematerial zu minimieren und den Verlust von gasförmigem Kohlenstoff in dem Biomassematerial zu minimieren;
c) Erzeugen eines Stoffwechselprodukts in Form von gasförmigem H₂ und/oder flüssigem 2,3-Butandiol aus dem Biomassematerial im Verhältnis zu Zeit- und Volumeneinheiten, ohne den tatsächlichen Zellwachstumsmetabolismus innerhalb des Biomassematerials zu optimieren;
d) Freisetzen mindestens eines ersten Teils des Stoffwechselprodukts in einen Verbrennungsprozess, um ein Verbrennungsprodukt zu erzeugen, und Einführen des Verbrennungsprodukts in das Trägergas vor dem Einführen in das Roh-Biomassematerial aus Abfällen; und
e) Auffangen mindestens eines zweiten gasförmigen Teils des Stoffwechselprodukts, der Wasserstoff enthält.

2. Verfahren nach Anspruch 1, **dadurch gekennzeichnet, dass** das mikrobiologische Herstellungsverfahren ein aus einem aeroben, einem mikroaeroben oder einem anaeroben Prozess ausgewählter Prozess ist.

3. Verfahren nach Anspruch 2, das ferner den Schritt des Regulierens mindestens eines unzuträglichen Zustands durch Zugabe von wenigstens einem von Glucose, einem Bakterium oder einer mikrobiellen Kultur zu der genannten Biomasse beinhaltet, wobei der unzuträgliche Zustand aus der aus reduziertem pH-Wert, erhöhtem pH-Wert und osmotischem Druck bestehenden Gruppe ausgewählt wird.

4. Verfahren nach Anspruch 3, das ferner den Schritt des Nutzens von mikrobiologischem Überflussmetabolismus zum Ansammeln des Stoffwechselprodukts oder zum Beschleunigen der Freisetzung des Stoffwechselprodukts beinhaltet.

5. Verfahren nach Anspruch 1, das ferner den Schritt des Einstellens der Temperatur des Trägergases vor der Einführung in das Roh-Biomassematerial umfasst.

6. Verfahren nach Anspruch 1, das ferner den Schritt des Konzentrierens des Stoffwechselprodukts in ein aus der Gruppe aus einer brennbaren Energiequelle, Motorkraftstoffen und einem Rohstoff der chemischen Industrie ausgewähltes umfasst.

7. Verfahren nach Anspruch 1, das ferner den Schritt des Umsetzens des Wasserstoffs nach Schritt e) mit Kohlenmonoxid zur Bildung von Methanol beinhaltet.

8. Verfahren nach Anspruch 1, das ferner den Schritt des Einleitens des Wasserstoffs aus Schritt e) in einen Verbrennungsturm beinhaltet.

## Revendications

1. Procédé de production microbiologique pour produire un produit métabolique sous la forme de H₂ gazeux et/ou de 2,3-butanediol liquide, ledit procédé comprenant les étapes :
a) d'introduction d'un gaz porteur dans une matière de biomasse de déchets bruts, ladite matière de biomasse contenant des *E.coli* et *Klebsiella mobilis,* et dans lequel le gaz porteur conduit dans la matière de biomasse brute est un gaz inerte, de l'azote, du bioxyde de carbone, du monoxyde de carbone, ou un mélange de ces gaz; **caractérisé en ce que** ledit procédé inclut en outre les étapes :
b) de fourniture d'un niveau de pH de la matière de biomasse de moins de pH 5 afin de minimiser le métabolisme de composés contenant du carbone dans la matière de biomasse et de minimiser la perte de carbone gazeux dans la matière de biomasse ;
c) de production d'un produit métabolique sous la forme de H₂ gazeux et/ou 2,3-butanediol liquide à partir de la matière de biomasse en fonction du temps et d'unités volumétriques sans optimiser le métabolisme de croissance cellulaire effectif au sein de la matière de biomasse ;
d) de libération d'au moins une première portion du produit métabolique vers un processus de combustion afin de produire un produit de combustion, et d'introduction du produit de combustion dans le gaz porteur avant son introduction dans la matière de biomasse de déchets bruts ; et
e) de capture d'au moins une deuxième portion gazeuse du produit métabolique contenant de l'hydrogène.

2. Procédé selon la revendication 1, **caractérisé en ce que** le procédé de production microbiologique est un processus sélectionné parmi un processus aérobie, microaérobie ou anaérobie.

3. Procédé selon la revendication 2 comprenant en outre l'étape de contrôle d'au moins une condition non avantageuse grâce à l'ajout à ladite biomasse d'au moins un élément parmi le glucose, une culture bactériene ou microbienne, la condition non avantageuse étant sélectionnée à partir du groupe consistant en un pH réduit, un pH accru, et une pression osmotique.

4. Procédé selon la revendication 3, incluant en outre l'étape d'exploitation d'un métabolisme de trop-plein microbien pour l'accumulation du produit métabolique, ou pour l'accélération de la libération du produit métabolique.

5. Procédé selon la revendication 1, incluant en outre l'étape de réglage de la température du gaz porteur avant son introduction dans la matière de biomasse brute.

6. Procédé selon la revendication 1, incluant en outre l'étape de concentration du produit métabolique en un élément sélectionné à partir du groupe composé d'une source d'énergie combustible, d'un carburant de moteur et d'une matière brute de l'industrie chimique.

7. Procédé selon la revendication 1 comprenant en outre l'étape de réaction de l'hydrogène après l'étape e) avec du monoxyde de carbone afin de créer du méthanol.

8. Procédé selon la revendication 1 comprenant en outre l'étape de conduction de l'hydrogène à partir de l'étape e) jusque dans une tour d'incinération.
